# EUROPÄISCHE PATENTSCHRIFT

(11) **EP 2 454 491 B1**
(45) Veröffentlichungstag und Bekanntmachung des Hinweises auf die Patenterteilung: **27.02.2019**
(21) Anmeldenummer: 10731464.3
(22) Anmeldetag: 03.07.2010
(51) Int. Cl.: F15B 19/00, F15B 21/04

(54) **MESSVORRICHTUNG NEBST HANDGERÄT ZUR BESTIMMUNG DER ELEKTRISCHEN SPANNUNG EINES STRÖMENDEN FLUIDS**
MEASURING APPARATUS TOGETHER WITH A HANDHELD INSTRUMENT FOR DETERMINING THE ELECTRICAL POTENTIAL OF A FLOWING FLUID
DISPOSITIF DE MESURE CONJOINTEMENT AVEC UN APPAREIL MANUEL, POUR LA DÉTERMINATION DE LA TENSION ÉLECTRIQUE D'UN FLUIDE EN ÉCOULEMENT

(30) Priorität: 17.07.2009 DE 102009033772
(43) Veröffentlichungstag der Anmeldung: 23.05.2012
(73) Patentinhaber: Hydac Filtertechnik GmbH, 66280 Sulzbach/Saar (DE)
(72) Erfinder: SCHMITZ, Andreas, 66459 Kirkel (DE); SCHWENDER, Matthias, 66459 Kirkel (DE); KOCH, Edwin, 66636 Tholey (DE); DUCHOWSKI, John Kazimierz, 66115 Saarbrücken (DE)
(74) Vertreter: Bartels und Partner, Patentanwälte
(86) Internationale Anmeldenummer: PCT/EP2010/004025
(87) Internationale Veröffentlichungsnummer: WO 2011/006590

(56) Entgegenhaltungen:
- DE-U1-202004 017 714
- JOHN K DUCHOWSKI ET AL: "OVERCOMING ELECTROSTATIC DISCHARGE IN HYDRAULIC AND LUBRICATING APPLICATIONS BY INCORPORATING A NOVEL FILTER MEDIA", , 1. Januar 2005 (2005-01-01), Seiten 1-9, XP007915807, Gefunden im Internet: URL:http://pall.tekgroup.com/pdf/10005/IFP E-Static_Charge.pdf [gefunden am 2010-11-16]

## Beschreibung

Die Erfindung betrifft eine Messvorrichtung zur Bestimmung der elektrischen, Spannung in einem Filtermedium und/oder in einem das Filtermedium durchströmenden Fluid mit den Merkmalen im Oberbegriff von Anspruch 1. Die Erfindung betrifft des Weiteren ein Verkaufsset für die Anwendung in der genannten Messvorrichtung nebst Handgerät.

Bauteile als Bestandteil von hydraulischen Anlagen, die von Fluid durchströmt werden, weisen bekanntermaßen verschiedenste Potentiale auf und bilden verschiedenste Kapazitäten aus. Insbesondere Filter mit einem Filtermedium, das im Betrieb zum Abreinigen von Fluid dient, sind als Bauteile hydraulischer Anlagen unter diesem Aspekt von Interesse. Durch den teilweise isolierenden Aufbau solcher Filter, bedingt durch Isolationsschichten zwischen filterwirksamen Schichten des Filtermediums oder durch eine Isolierung des Filtermediums gegenüber den Endkappen des Filters, sowie einem Stützrohr des Filters, kommt es beim Fluiddurchtritt mit eventuell vorliegender partikelartiger Verschmutzung zu elektrostatischen Aufladungen des Filtermediums und damit des Fluids selbst.

Aufgrund der erzeugten Potentialunterschiede, bedingt durch den Fluiddurchtritt, innerhalb des Filtermediums oder des Filters, der regelmäßig als austauschbares Filterelement in einem Filtergehäuse aufnehmbar ist, kann es dann zu plötzlichen Entladungen zwischen statisch aufgeladenen Filterelementeilen, wie beispielsweise des Filtermediums, kommen, gegenüber dem metallischen Filtergehäuse in dem das Filterelement aufgenommen ist, mit der Folge, dass eine Funkenbildung nicht ausgeschlossen werden kann. Dies ist bei brennbaren Fluiden, wie Hydrauliköl, Schweröl, Kraftstoffen oder dergleichen, grundsätzlich problematisch.

Zudem können solchen Funkenentladungen zu einer Schädigung des Öls und/oder des empfindlichen Filtermediummaterials führen. Ein vorzeitiges Altern von Öl verbunden mit einer Verkürzung der Wartungsintervalle für die Filterelementanordnung ist dann die Folge.

Um diesen Gegebenheiten entgegen zu wirken, kann es zweckmäßig sein, Gehäuse von Baukomponenten hydraulischer Anlagen zu erden, was aber nicht immer zu den erwünschten Resultaten führt und im Übrigen apparatetechnisch mit einem gewissen Aufwand verbunden ist. Hierzu können konstruktive Maßnahmen beim Filterbau vorgesehen sein, wie eine Erdung von Endkappen des Filterelementes über Elastomerdichtungen mit elektrischer Leitfähigkeit oder der Anordnung von Filtermaterialien mit unterschiedlichem Potential zum Potentialausgleich des durchströmenden Fluids im Filtermedium sowie Anordnung von Ladungsausgleichslagen, die die entstehende elektrische Ladung des Fluids zumindest teilweise an das zugeordnete Fluid zurückgeben können. Dergestalt können solche elektrostatischen Entladungen zumindest teilweise vermieden werden. Einen Aufschluss über die tatsächlichen elektrostatischen Verhältnisse in hydraulischen Anlagen, erhält man in der Regel allerdings auch bei einem Schadensfall, beispielsweise an einem Filterelement oder bei der Feststellung, dass eine rasche Alterung von Fluid, beispielsweise durch Alterungsprodukte im Öl, vorliegt.

Der Artikel Duchowski, John K.; Bensch, Leonard; Phair, Brendan; Khazan, Masha; Tsalyuk, Victor: "Overcoming Electrostatic Discharge in Hydraulic and Lubricating Applications By Incorporating A Novel Filter Media". In: NCFP I05-19.1, NFPA Technical Paper Series, vorgetragen auf der International Fluid Power Exposition, März 2005 - XP 007915807 - beschreibt eine Messvorrichtung zur Bestimmung der elektrischen Spannung in einem Filtermedium und/oder in einem das Filtermedium durchströmenden Fluid, mit einem Messgerät zur Messung von elektrischen Spannungswerten, an das eine Messeinrichtung anschließbar ist, die als Handgerät ausgebildet, zumindest einen Messkopf aufweist, der mit einem Fühler in das strömende Fluid für eine Messung eintaucht.

Ein Spannungsmesser geht aus der DE 20 2004 017 714 U1 hervor.

Ausgehend von diesem Stand der Technik liegt der Erfindung die Aufgabe zugrunde, eine Vorrichtung zu schaffen, mit deren Hilfe im Betrieb einer hydraulischen Anlage kritische elektrostatische Ladungen oder eine Entstehung von elektrischer Spannung in dem Fluid gemessen und insoweit aufgezeigt werden können.

Eine dahingehende Aufgabe löst eine Vorrichtung mit den Merkmalen des Patentanspruches 1.

Gemäß dem Kennzeichen von Anspruch 1 ist vorgesehen, dass der Messkopf mit dem Fühler eine Dichteinrichtung aufweist, die einen dichtenden Abschluss mit einer Anschlussstelle in einem Filtergehäuse bildet, das ein das Filtermedium tragendes Filterelement aufweist, dass der Messkopf mit dem Fühler über ein Verbindungsteil, in der Art eines Einschraubgewindes ausgebildet, mit der Anschlussstelle des Filtergehäuses lösbar verbunden ist und dass der Fühler des Messkopfes die Spannung des Fluids auf der Reinseite des Filtermediums abgreift.

Mittels der Dichteinrichtung lässt sich dergestalt eine dichtende Messanschlussverbindung zwischen der Messeinrichtung mit dem Handteil und dem Inneren des Filterelementgehäuses herstellen, so dass dergestalt ein ungewollter Austritt von Fluid aus dem Gehäuse selbst während des Filtrationsbetriebes vermieden ist, bei dem die Spannungsmessung vorzugsweise stattfindet.

Ferner hat es sich für die Messung als vorteilhaft erwiesen, mittels des Fühlers des Messkopfes die Spannung des Fluids auf der sogenannten Reinseite des Filtermediums abzugreifen, also auf der Seite, die sich aus der Abreinigung der Partikelverschmutzung mittels des Filtermediums ergibt.

Die erfindungsgemäße Messvorrichtung zur Bestimmung der elektrischen Spannung weist neben einem Messgerät zur Messung von elektrischen Spannungswerten eine an das Messgerät anschließbare Messeinrichtung auf, die als Handgerät ausgebildet, zumindest einen Messkopf beinhaltet, der mit einem Fühler in das strömende Fluid für eine Messung eintaucht. Dabei kann der in der Art einer Mess- oder Tastspitze ausbildbare Fühler auch in das durchströmte Filtermedium selbst eingreifen, ohne das aus Einzellagen bestehende Filtermedium zu beschädigen.

Eine Auswertung der gemessenen Spannungswerte erlaubt dann unter anderem Rückschlüsse über mögliche Potentialunterschiede zwischen Filtermedium des Filterelementes und dem zu reinigenden Fluid und es besteht dann dem Grunde nach die Möglichkeit solche Materialkombinationen für das Filtermedium auszuwählen, dass nach Möglichkeit ein geringer Potentialunterschied zwischen den eingesetzten Filtermaterialien und dem diese durchströmenden Fluid, insbesondere in Form von Hydrauliköl, geschaffen ist. Auch lässt sich gegebenenfalls durch Einsatz von Additiven im Fluid die triboelektrische Spannung zwischen Filtermedium und Fluid derart reduzieren, dass eben keine schädigenden Spannungseffekte während des Filtrationsbetriebes auftreten können.

Bei einer bevorzugten Ausführungsform der erfindungsgemäßen Messvorrichtung ist der genannte Fühler Bestandteil eines Hochspannungstastkopfes als Messkopf, der mit einer Isoliereinrichtung versehen, ein Handteil von dem eigentlichen Messteil mit dem Fühler separiert. Dergestalt ist ein sicherer Betrieb der Messvorrichtung möglich, indem eben eine Bedienperson der Messvorrichtung am Handteil angreifend die Messung vornimmt und dergestalt über die Isoliereinrichtung vor Spannungsüberschlägen geschützt ist.

Vorzugsweise ist dabei ferner vorgesehen, dass der Messkopf mit dem Fühler als eigenständige Baueinheit von dem Handteil mit der Isoliereinrichtung als weitere eigenständige Baueinheit separierbar ist. Dergestalt kann der Messkopf mit dem Fühler am jeweiligen Filterelementgehäuse verbleiben, so dass mit nur einem Handgerät, an das das Messgerät angeschlossen ist, eine Vielzahl im Betrieb befindlicher Filterelemente überprüft werden kann, ohne dass man für eine Messung den dahingehenden Filter außer Betrieb nehmen müsste.

Bei einer weiteren bevorzugten Ausführungsform der erfindungsgemäßen Messvorrichtung weist die Isoliereinrichtung einzelne Isolationsteile auf, die insbesondere als Isolierscheiben ausgebildet, einen Spannungsüberschlag von der Isoliereinrichtung auf das Handteil vermeiden helfen. Die einzelnen Isolationsteile verlängern den möglichen Überschlagweg, ausgehend vom Messkopf zum Handteil, was insoweit zur Erhöhung der Sicherheit für die Bedienperson mit beiträgt.

Um stabile Messwerte und eine niedrige Nachweisgrenze zu ermöglichen, weist der Fühler vorzugsweise Mittel zur Oberflächenvergrößerung auf, mit denen der Fühler in das strömende Fluid ragt. Die Mittel zur Oberflächenvergrößerung des Fühlers können neben Waben-, Porenstrukturen oder Steckmetallgittern bevorzugt in der Art einer Drahtbürste ausgebildet sein, wobei dann der Fühler als Teil des Messkopfes stiftartig sich von dem Messkopf weg erstreckt und entlang seines Stiftverlaufes mit zusätzlichen Teilfühlerelementen versehen ist, beispielsweise in der Art einer Borstenanordnung, bei der feine Drahtborsten sich von dem Fühlerstift nach außen hin weg erstrecken.

Vorzugsweise kann dabei ferner vorgesehen sein, dass der Fühler mit gruppenweise zueinander beabstandeten Drahtborsten in der Art einer Flaschenspülbürste versehen ist, wobei die Drahtborstengruppen dann einzelne scheibenartig voneinander getrennte Bereiche entlang des Messstiftverlaufes bilden. Bevorzugt sind die Drahtborsten dabei aus leitenden Materialien, wie aus Kupfer- oder Aluminiumwerkstoffen gebildet.

Für die Anwendung in einer Messvorrichtung kann ein Verkaufsset dienen, bestehend aus mindestens einem Handgerät mit Messkopf nebst Fühler und einem an das Handgerät anschließbaren Spannungs-Messgerät. Ein dahingehendes Verkaufsset, das die wichtigen Komponenten in einem Koffer oder dergleichen zusammenfasst, erlaubt dem Wartungspersonal eine problemlose Spannungsmessung vor Ort mit den Filteranlagen.

Da das eingesetzte Spannungs-Messgerät ein üblicher Voltmeter sein kann, der sich regelmäßig in Fertigungsstätten wiederfindet, kann es auch genügen, dem Bedien- und Wartungspersonal nur ein Handgerät zur Bestimmung der elektrischen Spannung in einem das Filtermedium durchströmenden Fluid zur Verfügung zu stellen, das dann nur aus einem Messkopf mit Fühler und einem hiervon mittels einer Isoliereinrichtung getrennten Handteil bestehen kann. Ist, wie dargelegt, der Messkopf mit Fühler an der jeweiligen Filtereinrichtung bereits vorhanden, kann es auch genügen, als Handgerät nur die Baueinheit, bestehend aus Isoliereinrichtung nebst Handteil, mitzuführen, wobei die Isoliereinrichtung nebst Handteil dann von mindestens einer Leiterbahn durchgriffen ist, die der Weiterleitung der Spannungswerte vom Messkopf zum eigentlichen Spannungs-Messgerät dient.

Da gegebenenfalls bei der Spannungsmessung auch sehr hohe Werte auftreten können, ist bevorzugt im Handteil ein kapazitiver handelsüblicher Spannungsteiler angeordnet, um so verwertbare Messspannungen an das Spannungs-Messgerät über die Verbindungsleitung weitergeben zu können. Des Weiteren ist die Isoliereinrichtung mit einem Massekabel versehen, das wiederum an das Spannungs-Messgerät anschließbar ist, das beispielsweise als handelsübliches digitales Multimeter zum Einsatz kommt. Anstelle eines dahingehenden digitalen Multimeters könnte auch ein Oszilloskop treten, wobei mit Hilfe des Oszilloskops als Spannungs-Messgerät es zusätzlich noch möglich wäre, elektromagnetische Spannungsspitzen oder Wellenverläufe als Interpretationshilfe darzustellen, die von der Funkenentladung im Bereich des Filterelementes ausgehen. Diese Entladungen sind dann als sogenannte Spannungsspitzen auf dem Display des Oszilloskops erkennbar und können insoweit für eine Gesamtauswertung ausgezählt werden.

Die genannte Messvorrichtung nebst Verkaufsset und Handmessgerät kann bei einem Kunden vor Ort auch dafür eingesetzt werden, zu zeigen, dass konventionelle Filterelementlösungen mit dem Phänomen der elektrostatischen Entladung einhergehen, und dass erst durch den Einsatz von speziell an das Fluid angepassten Filtermedien gemäss der Patentlehre der nachveröffentlichten DE 10 2008 004 344, sich eine Verbesserung ergibt, in dem Sinne, dass eben Potential unterschiede während des Filtrationsbetriebes vermieden sind, so dass insoweit auch keine unerwünschten Funkenentladungen mehr auftreten können.

Im Folgenden wird die erfindungsgemäße Lösung anhand der Zeichnung erläutert. Dabei zeigen in prinzipieller und nicht maßstäblicher Darstellung:
- Fig. 1: eine perspektivische Ansicht in der Art einer Explosionszeichnung auf eine Messeinrichtung für die Anwendung bei einer Filtereinrichtung, bestehend im Wesentlichen aus einem Filtergehäuse und einem darin aufgenommenen Filterelement; und
- Fig. 2: in vergrößerter Darstellung eine spezielle Ausführungsform eines Messfühlers gemäss der Darstellung nach der Fig. 1.

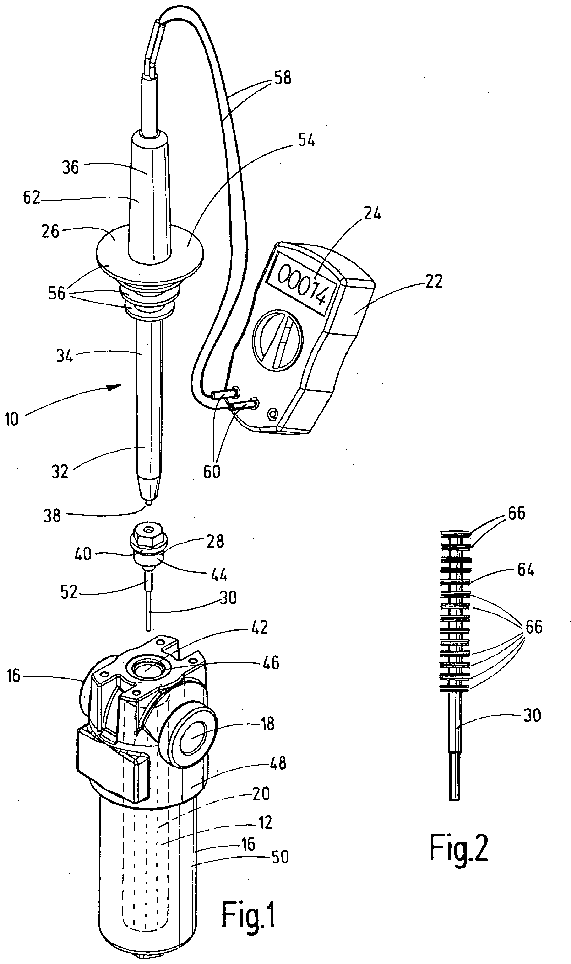

Die in Fig. 1 als Ganzes mit 10 bezeichnete Messvorrichtung dient der Bestimmung der elektrischen Spannung in einem Filtermedium 12 und/oder in einem das Filtermedium 12 durchströmenden Fluid. Das nicht näher dargestellte Fluid bevorzugt in Form eines Hydraulikmediums, wie Hydrauliköl, durchströmt über eine Einlassstelle 14 im Filtergehäuse 16 das Filtermedium 12 von außen nach innen und wird derart abfiltriert über eine Auslassstelle 18 aus dem Filtergehäuse 16 für die Weiterleitung in einen Hydraulikkreis (nicht dargestellt) abgeführt. Das Filtermedium 12 ist regelmäßig in Form einer plissierten Filtermatte ausgebildet, das in der Mitte von einem nicht näher dargestellten Stützrohr ausgesteift der Filtration des Fluids dient. Ein dahingehender Filterelementaufbau 20, im Wesentlichen bestehend aus dem sich am Stützrohr abstützenden Filtermedium 12, ist im Stand der Technik hinreichend bekannt, so dass an dieser Stelle hierauf nicht mehr näher eingegangen wird.

Insoweit trennt das Filterelement 20 innerhalb des Filtergehäuses 16 eine mit der Einlassstelle 14 verbundene Unfiltratseite von einer mit der Auslassstelle 18 verbundenen Reinseite. Etwaige Partikelverschmutzungen werden durch das Filtermedium 12 aus dem Fluid filtriert, wobei es bei dem dahingehenden Filtrationsvorgang zu elektrischen Potentialunterschieden kommen kann, die in Form von Funkenbildung sich schlagartig entladen können, was zu einer Schädigung des empfindlichen Filtermedienmaterials führen kann und im übrigen im Bereich brennbarer, zu filtrierender Fluidmengen grundsätzlich problematisch ist.

Zur Bestimmung der dahingehend auftretenden elektrischen Spannung weist die Messvorrichtung 10 ein Messgerät 22 auf, das ein handelsüblicher digitaler Multimeter sein kann; aber auch aus einem Oszilloskop (nicht dargestellt) als Messgerät bestehen kann. Das Messgerät 22 weist insbesondere eine Anzeigeeinrichtung 24 auf, zum Ablesen der ermittelten Spannungswerte innerhalb des Filtergehäuses 16, soweit die dahingehende Filtereinrichtung im Betrieb ist, d. h. zu filtrierendes Fluid die Filtereinrichtung durchströmt. Neben dem Messgerät 22 weist die Messvorrichtung 10 eine Messeinrichtung 26 auf, die, wie dargestellt, als Handgerät ausgebildet, einen separierbaren Messkopf 28 aufweist, der mit einem Messfühler 30 in das strömende Fluid für eine Messung eintaucht. Der genannte Fühler 30 ist Bestandteil eines Hochspannungstastkopfes 32, der mit einer Isoliereinrichtung 34 versehen, ein Handteil 36 von dem eigentlichen Messteil mit dem Fühler 30 trennt.

Der Messkopf 28 mit dem Fühler 30 weist eine Dichteinrichtung 40 in Form einer nach außen hin geschlossenen Dichtfläche auf, die einen dichtenden Abschluss mit einer oben angeordneten Anschlussstelle 42 in dem Filterelementgehäuse 16 bildet, das, wie bereits dargelegt, das Filtermedium 12 tragende Filterelement 20 aufweist. Der Messkopf 28 mit dem Fühler 30 wird dabei über ein Verbindungsteil, in der Art eines Einschraubgewindes 44 ausgebildet, mit der Anschlussstelle 42 des Filtergehäuses 16 lösbar verbunden, in die das Einschraubgewinde 44 als Außengewinde in Eingriff kommt mit einem Innengewinde 46 in der oberen Kopfaufnahme 48 des Filtergehäuses 16. Üblicherweise lässt sich die Kopfaufnahme 48 von dem sonstigen topfartigen Gehäuseteil 50 des Filtergehäuses 16 abschrauben, um dergestalt ein verbrauchtes Filterelement 20 gegen ein Neuelement zu tauschen. Auch die dahingehende Anordnung ist üblich, so dass an dieser Stelle hierauf nicht mehr näher eingegangen wird.

Die in Blickrichtung auf die Fig. 1 gesehen zuoberst angeordnete Anschlussstelle 42 des Filtergehäuses 16 dient üblicherweise der Aufnahme einer Filterverschmutzungsanzeige (nicht dargestellt), die bei Bedarf entsprechend entfernt die Anschlussstelle für den Messkopf 28 mit dem Messfühler 30 freigibt. Kann auf eine Verschmutzungsanzeige verzichtet werden oder kann diese gegebenenfalls an einer anderen Anschlussstelle im Gehäuse 16 angeordnet sein, besteht im Grunde nach die Möglichkeit eines permanenten Verbleibs des Messkopfs 28 mit dem Messfühler 30 am jeweiligen Filtergehäuse 16. Sofern also der Messkopf 28 mit dem Messfühler 30 als eigenständige Baueinheit 52 der sonstigen Messeinrichtung 26 ausgebildet ist, ist diese vom Handteil 36 mit der Isoliereinrichtung 34 als weitere eigenständige Baueinheit 54 separierbar. Dergestalt besteht grundsätzlich die Möglichkeit mit nur einer dahingehenden verbleibenden Messeinrichtung 26 eine Vielzahl von Filtereinrichtungen zu überprüfen, indem eben von außen her über ein Messanschlussteil 38 eine spannungsführende Verbindung mit dem Messfühler 30 hergestellt wird, indem die Messeinrichtung 26 nur in einfacher Weise von Hand auf die jeweils zu prüfende Filtereinrichtung aufgesetzt zu werden braucht. Dergestalt lässt sich zeitnah mit nur einer insoweit konzipierten Messeinrichtung 26 eine Vielzahl von Filtereinrichtungen, bestehend aus dem Filtergehäuse 16 mit dem jeweils aufgenommenen Filterelement 20, vor Ort prüfen.

Ein guter Messwertabgriff hat sich ergeben, sofern der Fühler 30 des Messkopfes 28 die Spannung des Fluids auf der Reinseite des Filterelements 20 erfasst. Um einen Spannungsüberschlag vom Messkopf 28 zum Handteil 36 der Messeinrichtung 26 vermeiden zu helfen, weist die Isoliereinrichtung 34 einzelne gestufte Isolierscheiben 56 auf, wie man sie in anderem Zusammenhang von Hochspannungsarmaturen bei Überlandleitungen her kennt.

Wie die Fig. 1 des weiteren zeigt, kann ein Verkaufsset für die Anwendung in der genannten Messvorrichtung 10 gebildet sein, bestehend aus einem Handgerät 62 mit Messkopf 28 nebst Fühler 30 und dem an das Handgerät 62 anschließbaren Spannungsmessgerät als Anzeigeeinrichtung 24. Für den dahingehenden Anschluss verlaufen zwischen der Anzeigeeinrichtung 24 und der Messeinrichtung 26 strom- oder spannungsführende Kabel 58, von denen ein Kabel bevorzugt als sogenanntes Massekabel ausgebildet ist. Der Anschluss der Kabel 58 an das Messgerät 22 kann über übliche Bananenstecker 60, wie dargestellt, erfolgen. Das Handgerät 62 zur Bestimmung der elektrischen Spannung in dem das Filtermedium 12 durchströmenden Fluid besteht, wie bereits dargelegt, mindestens aus dem Messkopf 28 mit Fühler 30 und dem mittels der Isoliereinrichtung 34 getrennten Handteil 36 zur sinnfälligen Bedienung von Hand mittels einer Bedienperson. Ferner kann bei einer bevorzugten Ausgestaltung der Messvorrichtung 10 bevorzugt innerhalb des Handteils 36 ein sogenannter Spannungsteiler angeordnet sein, der insbesondere bei hohen Messspannungen diese auf einen Spannungsmesswert begrenzt, der mit dem digitalen Multimeter störungsfrei für eine Auswertung auch angezeigt werden kann.

Wie die Fig. 2 darstellt, kann der an dem Messkopf 28 anschließbare Messfühler 30 zur Vergrößerung der wirksamen Messoberfläche mit Teilfühlerelementen 64 versehen sein, beispielsweise in Borstenanordnung, wobei die Einzelborsten, die in Borstenscheiben 66 voneinander getrennt sind, aus einem spannungsleitenden Material bestehen, beispielsweise aus einem Kupfer- oder Aluminiumwerkstoff.

Mit der erfindungsgemäßen Lösung ist eine schnelle, qualitative Einschätzung der Ölaufladungstendenz von Filterelementen 20 während des Filtrationsbetriebes möglich. Insbesondere wenn der Messfühler 30 in der Art einer Miniatur-Drahtbürste ausgebildet ist, wie dies sich aus der Fig. 2 ergibt, mit der die Spannung im Hydrauliköl abgenommen wird, lässt sich ein sehr feinfühliges Messinstrument erhalten, sofern die aufgezeigte Borstenanordnung im Ölstrom direkt hinter dem jeweiligen Filterelement 20, also auf der Reinseite, der Filtereinrichtung angeordnet ist. Mit der erfindungsgemäßen Messvorrichtung lässt sich vor Ort die elektrostatische Aufladung von bekannten Elementen darstellen und mit Hilfe innovativer Filtermattengestaltungen kann dem Phänomen der ungewollten elektrostatischen Entladung begegnet werden, sofern mittels des vorliegenden Handmessgerätes das Problem der Potentialunterschiede im Betrieb erkannt ist.

## Patentansprüche

1. Messvorrichtung (10) zur Bestimmung der elektrischen Spannung in einem Filtermedium und/oder in einem das Filtermedium (12) durchströmenden Fluid, mit einem Messgerät (22) zur Messung von elektrischen Spannungswerten, an das eine zur Messvorrichtung (10) gehörende Messeinrichtung (26) anschließbar ist, die als Handgerät ausgebildet, zumindest einen Messkopf (28) aufweist, der mit einem Fühler (30) in das strömende Fluid für eine Messung eintaucht, **dadurch gekennzeichnet, dass** der Messkopf (28) mit dem Fühler (30) eine Dichteinrichtung (40) aufweist, die einen dichtenden Abschluss mit einer Anschlussstelle (42) in einem Filtergehäuse (16) bildet, das ein das Filtermedium (12) tragendes Filterelement (20) aufweist, dass der Messkopf (28) mit dem Fühler (30) über ein Verbindungsteil, in der Art eines Einschraubgewindes (44) ausgebildet, mit der Anschlussstelle (42) des Filtergehäuses (16) lösbar verbunden ist und dass der Fühler (30) des Messkopfes (28) im Gebrauch die Spannung des Fluids auf der Reinseite des Filtermediums (20) abgreift.

2. Messvorrichtung nach Anspruch 1, **dadurch gekennzeichnet, dass** der Fühler (30) Bestandteil eines Hochspannungstastkopfes (32) ist, der mit einer Isoliereinrichtung (34) versehen, ein Handteil (36) von dem Messteil (38) mit dem Fühler (30) separiert.

3. Messvorrichtung nach Anspruch 2, **dadurch gekennzeichnet, dass** der Messkopf (28) mit dem Fühler (30) als eigenständige Baueinheit (52) von dem Handteil (36) mit der Isoliereinrichtung (34) als weitere eigenständige Baueinheit (54) separierbar ausgestaltet ist.

4. Messvorrichtung nach Anspruch 2 oder 3, **dadurch gekennzeichnet, dass** die Isoliereinrichtung (34) einzelne Isolationsteile aufweist, die insbesondere als Isolierscheiben (56) ausgebildet, einen Spannungsüberschlag von der Isoliereinrichtung (34) auf das Handteil (36) vermeiden helfen.

5. Messvorrichtung nach einem der Ansprüche 1 bis 4, **dadurch gekennzeichnet, dass** zur Vergrößerung der wirksamen Messoberfläche des Fühlers (30) dieser mit Teilfühlerelementen (64), vorzugsweise in Borstenanordnung (66), versehen ist.

## Claims

1. A measuring device (10) for determining the electrical voltage in a filter medium and/or in a fluid flowing through the filter medium (12), with a measuring instrument (22) for measuring electrical voltage values, to which it is possible to connect a measuring apparatus (26), which belongs to the measuring device (10) and which is formed as a hand-held instrument, and has at least one measuring head (28) which dips into the flowing fluid with a sensor (30) for a measurement, **characterised in that** the measuring head (28) with the sensor (30) has a sealing device (40) which forms a sealing connection with a connection point (42) in a filter housing (16) which has a filter element (20) bearing the filter medium (12), **in that** the measuring head (28) with the sensor (30) is detachably connected to the connection point (42) of the filter housing (16) via a connecting part designed in the manner of a thread (44), and **in that**, when in use, the sensor (30) of the measuring head (28) taps the voltage of the fluid on the clean side of the filter medium (20).

2. The measuring device according to claim 1, **characterised in that** the sensor (30) is a component of a high voltage probe head (32) which is provided with an insulating apparatus (34) which separates a hand part (36) from the measuring part (38) with the sensor (30).

3. The measuring device according to claim 2, **characterised in that** the measuring head (28) with the sensor (30) is designed separable as independent structural unit (52) of the hand part (36) with the insulating apparatus (34) as a further independent structural unit (54).

4. The measuring device according to claim 2 or 3, **characterised in that** the insulating apparatus (34) has individual insulating parts which, in particular designed as insulating disks (56), help prevent a voltage flashover from the insulating apparatus (34) to the hand part (36).

5. The measuring device according to one of claims 1 to 4, **characterised in that**, to enlarge the effective measuring surface of the sensor (30), this is provided with partial sensor elements (64), preferably in bristle arrangement (66).

## Revendications

1. Système (10) de mesure pour déterminer la tension électrique dans un milieu filtrant et/ou dans un fluide passant à travers le milieu (12) filtrant, comprenant un appareil (22) de mesure pour mesurer des valeurs électriques de tension, auquel peut être raccordé un dispositif (26) de mesure, qui appartient au système (10) de mesure et qui, constitué en appareil à main, a au moins une tête (28) de mesure, qui, pour une mesure, plonge dans le fluide en écoulement, **caractérisé en ce que** la tête (28) de mesure, ayant la sonde (30), a un dispositif (40) d'étanchéité, qui forme une fermeture étanche avec un point (42) de raccordement dans une enveloppe (16) de filtre, qui a un élément (20) de filtre portant le milieu (12) filtrant, **en ce que** la tête (28) de mesure, ayant la sonde (30), est reliée de manière amovible, par une partie de liaison constituée à la manière d'un filetage (44) de vis, du point (42) de raccord de l'enveloppe (16) de filtre et **en ce que** la sonde (30) de la tête (28) de mesure prélève, en utilisation, la tension du fluide du côté propre du milieu (20) filtrant.

2. Système de mesure suivant la revendication 1, **caractérisé en ce que** la sonde (30) fait partie d'une tête (32) de balayage de haute tension, qui, pourvue d'un dispositif (34) isolant, sépare une partie (36) à main de la partie (38) de mesure ayant la sonde (30).

3. Système de mesure suivant la revendication 2, **caractérisé en ce que** la tête (28) de mesure, ayant la sonde (30), est conformée, avec possibilité de séparation, sous la forme d'une unité (52) de construction indépendante de la partie (36) à main ayant le dispositif (34) isolant, comme autre unité (54) de construction indépendante.

4. Système de mesure suivant la revendication 2 ou 3, **caractérisé en ce que** le dispositif (34) isolant a des parties isolantes individuelles, qui, constituées notamment sous la forme de rondelles (56) isolantes, contribuent à empêcher une décharge de tension du dispositif (34) isolant à la partie (36) à main.

5. Système de mesure suivant l'une des revendications 1 à 4, **caractérisé en ce que**, pour augmenter la surface efficace de mesure de la sonde (30), celle-ci est pourvue d'éléments (64) de sonde partiels, de préférence suivant un agencement (66) à soies.
